# EUROPÄISCHE PATENTSCHRIFT

(11) **EP 1 408 968 B1**
(45) Veröffentlichungstag und Bekanntmachung des Hinweises auf die Patenterteilung: **23.11.2005**
(21) Anmeldenummer: 02753025.2
(22) Anmeldetag: 18.07.2002
(51) Int. Cl.: A61K 31/46, A61K 31/415, A61K 9/70

(54) **REZEPTORADAPTIERTER NIKOTINENTZUG DURCH EINE KOMBINATION VON ATROPIN, SCOPOLAMIN, CLONIDIN**
RECEPTOR-ADAPTED NICOTINE WITHDRAWAL BY A COMBINATION OF ATROPINE, SCOPOLAMINE, CLONIDINE
SEVRAGE A LA NICOTINE ADAPTE AUX RECEPTEURS PAR UNE COMBINAISON D'ATROPINE, SCOPOLAMINE, CLONIDINE

(30) Priorität: 25.07.2001 DE 10136228
(43) Veröffentlichungstag der Anmeldung: 21.04.2004
(73) Patentinhaber: Kox, Wolfgang J., 14129 Berlin (DE); Hensel, Mario, 13089 Berlin (DE)
(72) Erfinder: KOX, Wolfgang J., 14129 Berlin (DE); HENSEL, Mario, 13089 Berlin (DE)
(74) Vertreter: Bergmann, Simon
(86) Internationale Anmeldenummer: PCT/DE2002/002626
(87) Internationale Veröffentlichungsnummer: WO 2003/015782

(56) Entgegenhaltungen:
- EP-A- 1 072 260
- WO-A-00/74651
- US-A- 4 788 189
- US-A- 6 004 970
- US-A- 6 132 754
- GLASSMAN A H ET AL: "Cigarette craving, smoking withdrawal, and clonidine." SCIENCE. UNITED STATES 16 NOV 1984, Bd. 226, Nr. 4676, 16. November 1984 (1984-11-16), Seiten 864-866, XP009007719 ISSN: 0036-8075
- HILLEMAN DANIEL E ET AL: "Randomized, controlled trial of transdermal clonidine for smoking cessation." ANNALS OF PHARMACOTHERAPY, Bd. 27, Nr. 9, 1993, Seiten 1025-1028, XP009008022 ISSN: 1060-0280
- PROCHAZKA ALLAN V ET AL: "Transdermal clonidine reduced some withdrawal symptoms but did not increase smoking cessation." ARCHIVES OF INTERNAL MEDICINE, Bd. 152, Nr. 10, 1992, Seiten 2065-2069, XP009008021 ISSN: 0003-9926
- NIAURA RAYMOND ET AL: "Transdermal clonidine for smoking cessation: A double-blind randomized dose: Response study." EXPERIMENTAL AND CLINICAL PSYCHOPHARMACOLOGY, Bd. 4, Nr. 3, 1996, Seiten 285-291, XP009008023 ISSN: 1064-1297

## Beschreibung

Die Erfindung betrifft die kombinierte Anwendung anticholinerg und antinoradrenerg wirksamer Substanzen zur Behandlung des Nikotin-Entzugssyndroms (NES), insbesondere gemäß dem Oberbegriff der Ansprüche 1 und 6. Die Erfindung führt zur Blockade einer überschüssigen Acetylcholin- bzw. Noradrenalinfreisetzung in der initialen Phase des Nikotinentzuges und damit zu einer Reduktion der Entzugssymptomatik.

Seit die Seefahrer um Christoph Columbus nach ihrer Rückkehr aus Amerika den Tabak in die zivilisierte Welt eingeführt haben, hat sich der Nikotinabusus zur weltweit häufigsten Form des Substanzmissbrauchs entwickelt. Er ist legal, wird durch die Werbeindustrie gefördert und ist in weiten Teilen der Gesellschaft akzeptiert. Raucher verbinden mit dem Gebrauch der Zigarette angenehme Begleiterscheinungen wie den Abbau von Spannungen und die Erhöhung der Konzentrationsfähigkeit. Allerdings hat das Zigarettenrauchen erhebliche volkswirtschaftliche, gesundheitspolitische und soziale Auswirkungen. Die durch Nikotinabusus verursachte Morbidität und Mortalität ist höher als bei allen anderen Drogen zusammen (Milei J, Grana DR, Int J Cardiol 67 (1998) 95-109). Die Rückfallquote bei kurzzeitig abstinenten Rauchern liegt bei ca. 75% (Evan RI et al., Atherosclerosis Reviews 6 (1979) 201-241.).

Nikotin beeinflusst vor allem das Zentralnervensystem, wobei die Abstinenz vom Nikotin Entzugssymptome wie Nervosität und Reizbarkeit verursacht, was einen Nikotinentzug so unangenehm macht. Beim Nikotinabusus existiert, ebenso wie bei anderen suchterzeugenden Substanzen, sowohl eine physiologische (biochemische) als auch eine psychologische (gewohnheitsmäßige) Komponente der Abhängigkeit. Herkömmliche Entzugsverfahren sind vor allem auf die Behandlung der psychischen Abhängigkeit orientiert, welcher sicherlich eine große Bedeutung beizumessen ist. Zunächst muss jedoch die physische Abhängigkeit überwunden werden, um dann die Gewohnheiten erfolgreich ändern zu können. Therapiekonzepte, die sich der suchterzeugenden Substanz selbst bedienen (Nikotinpflaster, Nikotinkaugummi), erscheinen ungeeignet, weil diese Verfahren die Suchtmechanismen auf der Ebene der Neurotransmitter nicht blockieren, sondern aufrechterhalten. Darüber hinaus sind eine Reihe anderer Methoden beschrieben worden, so z.B. Hypnose, Psychotherapie, Elektroschock-Aversionstherapie und Gruppentherapien (E-van RI et al., Atherosclerosis Reviews 6 (1979) 201-241). Großangelegte Studien haben jedoch zeigen können, dass weniger als die Hälfte aller Raucher, die an solchen Programmen teilnehmen, den Zigarettenkonsum konsequent abbrechen und nur 25-30% nach einem Zeitraum von 9-18 Monaten noch Nichtraucher sind (Stapleton J., Stat Methods Med Res 7 (1998) 187-203).

Sowohl für die Entstehung der Nikotinabhängigkeit, als auch für die Ausprägung des NES spielt der Neurotransmitter Acetylcholin (ACH) eine wichtige Rolle (Wessler 1, Kirkpatrick Ch J., Deutsches Ärzteblatt 97 (2000) B41-B42.). ACH wirkt an zentralen und peripheren (autonom, motorisch) cholinergen Neuronen durch Stimulation von Nikotin- und Muskarinrezeptoren. Es ist gut dokumentiert, dass Nikotin eine agonistische Wirkung auf die Nikotinrezeptoren des parasympathischen Nervensystems, aber auch auf muskarinerge Rezeptoren, besitzt (Levin ED, Simon BB, Psychopharmacology-Berl 138 (1998) 217-2305). Bei Langzeitexposition wirkt Nikotin primär als blockierende Substanz. Obwohl diese Wirkung weniger für das zentrale als für das periphere Nervensystem beschrieben wurde, scheint sich der Hauptlokalisationsort der Nikotinrezeptoren im Mittelhirn zu befinden (Bachynsky N., Int J Addict 21 (1986) 789-805). Durch den chronischen Nikotingebrauch kommt es zur Entwicklung von Toleranz und später zur Abhängigkeit. Diese Anpassungsphänomene basieren auf biochemischen Veränderungen an spezifischen Rezeptoren des parasympathischen Nervensystems, einhergehend mit einer Akkumulation von Acetylcholin. Studien an Wirbeltieren haben gezeigt, dass die dauerhafte Nikotin-Exposition cervikaler Ganglien zu einer um 35% höheren ACH-Ausschüttung führten (Levin ED, Simon BB, Psychopharmacology-Berl 138 (1998) 217-2305). Demgegenüber kam es unmittelbar nach Beendigung der Nikotin-Exposition zu erheblichen Neurotransmitterveränderungen, die zu einer Verminderung der ACH-Konzentrationen bis hin zu subnormalen Spiegeln, führten. Gleichzeitig wurde eine Normalisierung der Aktivität der Cholin-Acetyltransferase (Acetylcholinsynthese) und eine erhöhte Aktivität der ACH-Esterase (Acetylcholinabbau) beobachtet. Diese Effekte sind auf die fehlende Nikotinstimulation zurückzuführen. Allerdings kommt es reaktiv zu einer erhöhten ACH-Freisetzung aus zentralen neuronalen Strukturen sowie aus peripheren Nervenendigungen (Levin ED, Simon BB, Psychopharmacology-Berl 138 (1998) 217-2305). Somit entsteht durch den Wegfall der nikotinergen Blockade an den spezifischen nikotinerg-cholinergen Synapsen ein "Nikotin-Entzugssyndrom". Toleranz und Abhängigkeit, die durch eine erhöhte ACH-Synthese charakterisiert sind, werden durch Entzugsphänomene ersetzt, welche auf eine excessive Stimulation durch ACH zurückzuführen sind. Letztlich ist das Nikotin-Entzugssyndrom biochemisch als reaktives "ACH-Bombardement" nikotinerger Rezeptoren auf der unteren Mittelhirnebene zu interpretieren. Dies wird wiederum durch die synaptische Interaktion mit muskarinergen Rezeptoren, die auf höherer neuronaler Ebene, z.B. dem Cortex, stimuliert wurden, ausgelöst (Bachynsky N., Int J Addict 21 (1986) 789-805).

Körperliche Symptome, die im akuten Nikotinentzug auftreten sind reduzierte Herzfrequenz, verminderter Blutdruck, erhöhte Reizbarkeit, Nervosität, Störungen der Magen-Darm-Funktion, EEG-Veränderungen und verminderte Konzentrationsfähigkeit (Hays JT et al., Postgrad Med 104 (1998) 56-62). Diese Symptomatik entspricht dem klinischen Bild bei einem erhöhten Parasympathikotonus. Als Reaktion auf das Entzugssyndrom nimmt der abhängige Raucher den Zigarettenkonsum wieder auf, um sich mit Hilfe des Nikotin an seinen biochemischen "Normalzustand" "heranzutitrieren". Glick, Jarvick und Nakamura testeten eine Reihe anticholinerger und anderer Substanzen an Affen und fanden heraus, dass Scopolamin und d-Amphetamin zu einer Verminderung rauchertypischer Verhaltensmuster führten (Glick SD et al., Nature 227 (1970) 969-971).

Bachynsky und Mitarbeiter versuchten durch die subkutane, aurikuläre Injektion einer Atropin-/Scopolaminlösung, ergänzt durch Chlorpromazin, eine excessive cerebrokortikale ACH-Stimulation zu verhindern (Bachynsky N., Int J Addict 21 (1986) 789-805). Auf diese Weise sollten Entzugssymptome, die durch den Wegfall der chronischen Nikotinexposition entstehen, vermindert werden. Die US 4,555,397 beschreibt dieses Verfahren. Insoweit wurde über die klinischen Effekte beschrieben, dass aurikuläre Injektionen einen prompten Effekt im Sinne einer Symptomreduktion brachten. Da im Nikotinentzug über Tage und Wochen erhöhte ACH-Spiegel nachweisbar sein können, wurden die Patienten nach der initialen therapeutischen Intervention durch orale Medikation auf zentral wirksame anticholinerge Substanzen eingestellt. Während 2 Monate nach dem Entzug noch zwischen 66% (ohne Chlorpromazin behandelt) und 87% (mit Chlorpromazin behandelt) von den 500 therapierten Patienten abstinent lebten, waren es nach 12 Monaten nur noch 19% bzw. 39%.

Neben den Imbalancen des cholinergen Transmittersystems kommt es während des Nikotinentzuges v.a. zu Ungleichgewichten des noradrenergen Systems. In diesem Zusammenhang hat sich die Anwendung des Alpha-2-Agonisten Clonidin als erfolgversprechende Therapieoption erwiesen (Toon S., Eur J Pharm Biopharm 41 (1995) 184-188). Clonidin vermindert in bestimmten Hirnstrukturen, wie dem *Locus coeruleus,* die Noradrenalinfreisetzung. Glassman et al. setzten in einer randomisierten, plazebokontrollierten Studie Clonidin bei Vorliegen einer schweren Nikotinabhängigkeit (> 60 Zigaretten pro Tag) ein und fanden in der Therapiegruppe einen um 50% höheren Behandlungserfolg (Abstinenz), als in der Kontrollgruppe (Glassman P., JAMA 259 (1988) 2863-2866). Gourlay et al. konnten in einer Dosisfindungsstudie zeigen, dass mit steigender Clonidindosierung auch der Anti-Craving-Effekt (suchtdruckmindernde Wirkung) zunahm (Gourlay W., Clin Pharmacol Ther 55 (1994) 64-69).

Da die Symptome des Nikotinentzuges in den ersten 24-48 Stunden am ausgeprägtesten sind, ist eine möglichst schnelle Anflutung sowie initial hohe Plasmaspiegel der anticholinergen und antinoradrenergen Substanzen notwendig. Obwohl das Nikotin ca. 3 Tage nach Abstinenzbeginn vollständig aus dem Körper eliminiert ist, dauern die Entzugssymptome bis zu 2 Wochen an (Bachynsky N., Int J Addict 21 (1986) 789-805). Mit dieser klinischen Erfahrung stimmen auch Laboruntersuchungen überein, die zeigen konnten, dass die erhöhte ACH-Synthese erst nach ca. 14 Tagen rückläufig ist (Bachynsky N., Int J Addict 21 (1986) 789-805). Erhöhte Plasmakatecholaminspiegel können ebenfalls über Tage nachgewiesen werden. Als Konsequenz aus dieser Tatsache müsste eine Dauermedikation über einen Zeitraum von 2-3 Wochen erfolgen, die eine adäquate anticholinerge und antinoradrenerge Aktivität aufrechterhält. Außerdem hilft die protrahierte anticholinerge Blockade dem Patienten durch Veränderungen des Geruchs- und Geschmacksinns eine Aversion gegen das Zigarettenrauchen zu entwickeln. Während die medikamentenbedingte Mundtrockenheit in anderen Situationen unerwünscht ist, wirkt sie bei dieser Methode als unterstützender Faktor für den Behandlungserfolg.

Durch Medikamenten-Applikationen per Injektion können die gewünschten Pharmakawirkungen nur vorübergehend erreicht werden. Auch der orale Zugangsweg ist mit begrenztem Patientenkomfort und mit diskontinuierlichen Blutplasmaspiegeln verbunden. Demgegenüber besitzen transkutane therapeutische Systeme (TTS) die o.g. Eigenschaften am ehesten. Solche Systeme haben sich beispielsweise bei der Behandlung des Bluthochdrucks, zur kontinuierlichen Hormonsubstitution und zur Behandlung der Reisekrankheit bewährt.

Im relevanten Stand der Technik diskutiert die US 6,132,754 A eine Therapie zur Entwöhnung von Tabak- und Nikotinabhängigkeit. Dabei wird eine Kombination von Atropin und Scopolamin zur Injektion zum Nikotinentzug beansprucht.

Die WO 001074651 offenbart die intranasale Anwendung von anticholinergen Wirkstoffen wie beispielsweise Atropin oder Scopolamin. Die Kombination der beiden beispielhaft genannten Wirkstoffe zur intranasalen Applikation offenbart diese Druckschrift jedoch nicht.

Glassman et al. (Science Bd. 226, Nr. 4676, 1984, S. 864-866) zeigen in ihrer Studie eine Reduktion der Symptome des Nikotinentzuges durch Clonidin. Dabei demonstrieren sie, dass die noradrenerge Aktivität ein gemeinsames Merkmal der Pathophysiologie des Entzuges ist und eine spezielle Verbindung zwischen zentraler moradrenerger Aktivität und Sucht besteht.

In der US 4,788,189 A wird eine Methode zur Behandlung von Zigarettenentzugs-Symptomen beschrieben, die beispielsweise eine initiale Gabe von oralem Clonidin-HCl (0,1 mg) zusammen mit einem weiteren Wirkstoff (Impramin) vorsieht.

Die EP 1 072 260 offenbart Transdermalsysteme zur Abgabe von Muscarin-Rezeptor-Antagonisten und deren Verwendung zur Behandlung von Spasmen der glatten Muskulatur im urologischen Bereich. Bei den offenbarten Muscarin-Rezeptor-Antagonisten handelt es sich um ein oder mehrere Verbindungen, wie auch Atropin oder Scopolamin.

In der US 6,004,970 werden die vielen Nebenwirkungen einer oralen Gabe von Clonidin diskutiert, weshalb in dieser Druckschrift transdermale Systeme zur Applikation untersucht wurden. Dabei wird auf frühere Ergebnisse verwiesen, die ergeben haben, dass transdermal appliziertes Clonidin den Nikotinentzug mildert, jedoch letztlich nicht dazu führten, dass eine größere Anzahl an Personen das Rauchen aufgab.Auch wenn in diesem Dokument die Gabe von Clonidin sowie Clonidin Dosierungen diskutiert wird, befasst sich diese Druckschrift im wesentlichen mit der Behandlung der Nikotinabhängigkeit mit Opioid-Antagonisten.

Die Studie von Niaura Raymond et al. (Experimental and clinical Psychopharmacology, Bd. 4, Nr. 3, 1996, S. 285-291) befasst sich ebenfalls mit der transdermalen Applikation von Clonidin zur Raucherentwöhnung. Dabei wird ausschließlich die alleinige Gabe von Clonidin und deren Effekt untersucht.

Ausgehend von diesem Stand der Technik ist es die Aufgabe der vorliegenden Erfindung, pharmazeutisch wirksame Mittel (Arzneimittel) zur Verfügung zu stellen, die effizient und nachhaltig den Nikotinentzug fördern und unterstützen, sowie geeignete Applikationsmethoden zu schaffen.

Gelöst wird diese Aufgabe durch die Merkmale der unanhängigen Ansprüche.

Dabei ist es erfindungsgemäß vorgesehen, dass zwei unterschiedliche Wirkstoffkombinationen (vorzugsweise als Vakzine) verwendet werden, die zum einen eine wirksame Menge einer Kombination von Atropin und Scopolamin (bevorzugt jeweils 0,1 mg) und zum anderen eine wirksame Menge einer Kombination von Atropin, Scopolamin und Clonidin (bevorzugt bei 0,1 mg Atropin und Scopolamin) und Clonidin (bevorzugt 0,15 mg) enthält.

Um die Vorzüge transkutaner therapeutischer Systeme bei der Behandlung des Nikotin-Entzugssyndroms nutzen zu können, wurden erfindungsgemäß Hautpflaster konzipiert, die mit anticholinergen und antinoradrenergen Substanzen beschichtet werden, nämlich mit Clonidin, Scopolamin und/oder Atropin. Zur Steigerung der Effizienz der Pflasteranwendung geht eine einmalige intramuskuläre Injektion der genannten Substanzen voraus, mit dem Ziel, initial hohe Plasmaspiegel zu schaffen. Hierzu wurden erfindungsgemäß geeignete Vakzine geschaffen, die eine wirksame Menge einer Kombination von Atropin und Scopolamin bzw. Atropin, Scopolamin und Clonidin enthalten.

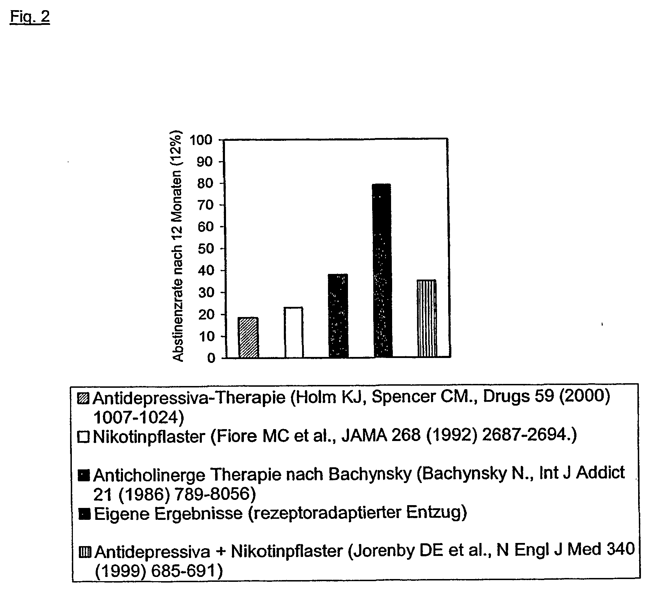

Mit den erfindungsgemäßen Maßnahmen lassen sich überraschenderweise um bis zu 50% bessere Erfolge erzielen, als mit herkömmlichen Methoden (6, 12, 13, 14; siehe Figur 2)

Weitere vorteilhafte Maßnahmen sind in den übrigen Unteransprüchen beschrieben; die Erfindung wird anhand eines Ausführungsbeispiels und von Figuren nunmehr näher beschrieben.

Im folgenden wird ein Therapieplan zum Nikotinentzugsverfahren beschrieben, in dessen Rahmen die erfindungsgemäße Medikation eingesetzt wurde:

Bevor das Nikotinentzugsverfahren durchgeführt werden kann, muss der Patient einen Fragebogen über die Gewohnheiten als Raucher ausfüllen und wichtige anamnestische Daten liefern. Besonderes Augenmerk wird dabei auf das Vorliegen potentieller Kontraindikationen gerichtet. Außerdem wird vermerkt, ob es in der Vergangenheit paradoxe Reaktionen oder Nebenwirkungen infolge der Anwendung anticholinerger oder antinoradrenerger Substanzen bzw. Allergien gegen diese Substanzen gab. Patienten, bei denen Kontraindikationen vorliegen, dürfen nach diesem Verfahren nicht behandelt werden.

Die Behandlung selbst beginnt mit einem Beratungsgespräch. Dabei wird zunächst die Therapie detailliert beschrieben und die medizinischen Grundlagen werden erläutert. Außerdem werden Empfehlungen zu Verhaltensänderungen gegeben. Nach einer Reihe von klinischen Untersuchungen werden den Patienten durch 2 intramuskuläre Injektionen insgesamt 0,2 mg Atropin, 0,2 mg Scopolamin und 0,15 mg Clonidin verabreicht. Für diese Injektionen werden vorgefertigte Einwegspritzen oder aber Fertigspritzen mit Hilfe automatischer Injektorensysteme genutzt. Eine Injektion wird mittels einer Vakzine durchgeführt, die Atropin und Scopolamin bevorzugt in einer Menge von jeweils 0,1 mg enthält; die weitere Injektion wird mittels einer Vakzine durchgeführt, die Atropin und Scopolamin bevorzugt in einer Menge von jeweils 0,1 mg und Clonidin bevorzugt in einer Menge von 0,15 mg enthält.

Am darauffolgenden Tag wird die Kontinuität der Behandlung mit transkutanen therapeutischen Systemen fortgesetzt. Dazu werden 2 Pflaster auf verschiedene Hautpartien aufgeklebt (z.B. im Bereich der Oberarme) (1 x Clonidin-Pflaster, 1 x Atropin / Scopolamin-Pflaster). Bei geeigneten Kandidaten werden durch die anticholinerge und noradrenerge Blockade nicht nur die körperlichen Entzugssymptome verhindert, sondern es wird auch die psychische Komponente der Nikotinsucht ("Craving") reduziert. Die Medikamentendosis auf den Hautpflastem wird den individuellen Bedürfnissen der Patienten angepasst. Wenn schon mehrere Entzugsversuche erfolglos abgebrochen wurden oder wenn über besonders ausgeprägte Entzugssymptome berichtet wird, so kann die Dosis erhöht werden. Eine Verwendung eines Hautpflasters mit zwei separaten Kontaktflächen, die jeweils ein Clonidin-Pflaster bzw. Atropin / Scopolamin-Pflaster darstellen, ist ebenfalls bevorzugt.

Die Dosierungsbereiche liegen beim Atropin und Scopolamin zwischen 0,5 und 1,5 mg (Maximaldosierung) sowie beim Clonidin zwischen 0,1 und 0,5 mg (Maximaldosierung). Die bevorzugte Dosierung enthält 1 mg Atropin, 1 mg Scopolamin und 0,3 mg Clonidin. Die Absorptionsfläche für Atropin-/Scopolamin-Membranpflaster beträgt 0,8 - 2,5 cm² und für Clonidin- Membranpflaster 1,2 - 3 cm².

Typischerweise kommt es durch die Anticholinergikagabe zu leichten Sehstörungen mit erhöhter Lichtempfindlichkeit und Akkomodationsstörungen. Diese Effekte verschwinden nach 6 bis 8 Stunden. Außerdem kann es sein, dass die Patienten über Geruchs- und Geschmacksveränderungen klagen, die sich jedoch nach ca. 24 Stunden auflösen. Die Clonidingabe kann mit hypotonen Kreislaufreaktionen einhergehen. Deswegen sollte stets auf eine genügende Flüssigkeitszufuhr geachtet werden. Die Patienten werden darüber aufgeklärt, dass während der Behandlung absolutes Alkoholverbot besteht. Außerdem wird darauf hingewiesen, dass Medikamente mit synergistischen Wirkungen, wie z.B. Sedativa, gemieden werden sollten.

Um die Umstellung des Nervensystems weiterhin pharmakologisch zu begleiten, müssen die Pflaster täglich gewechselt werden, wobei die Dosierung schrittweise und in individueller Absprache reduziert wird.

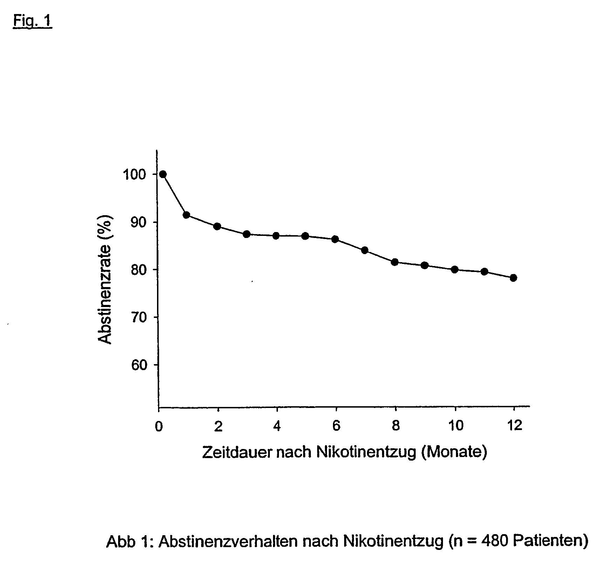

Die Erfindung führt zu dem überraschenden und nicht erwarteten Ergebnis, dass die Effektivität des Nikotinentzugs im Vergleich zu herkömmlichen Methoden signifikant erhöht werden kann: 480 Patienten wurden mit der beschriebenen Methodik und den erfindungsgemäßen Maßnahmen behandelt. Von diesen waren nach einem Zeitraum von 12 Monaten immerhin noch 78% abstinent (siehe Figur 1). Im Vergleich zu den von Bachynsky (Bachynsky N., Int J Addict 21 (1986) 789-805) publizierten Ergebnissen liegt die Erfolgsquote damit um nahezu 50% höher. Die wesentlichen Unterschiede zur Methode von Bachynsky sind wie folgt:

In der Figur 2 ist die überraschende und aufgrund der vorpublizierten Ergebnisse (Holm KJ, Spencer CM., Drugs 59 (2000) 1007-1024; Fiore MC et al., JAMA 268 (1992) 2687-2694; Jorenby DE et al., N Engl J Med 340 (1999) 685-691) nicht zu erwartende Wirkung der erfindungsgemäßen Maßnahmen dargestellt. Anhand der graphischen Darstellung ist unzweifelhaft zu erkennen, dass nur die erfindungsgemäßen Maßnahmen (in der Figur 2 als "eigene Ergebnisse" gekennzeichnet) einen langfristigen Erfolg beim Nikotinentzug gewährleisten, der siginfikant höher ist als bei vergleichbaren Anwendung von Bachynsky et al., Holm et al., Fiore et al. und Jorenby et al..

Die Anwendung transkutaner therapeutischer Systeme ermöglicht eine sichere, kontinuierliche und komfortable Blockade der Entzugssymptomatik über Wochen und ggf. auch Monate hinweg. Dabei werden Transmitterimbalancen des noradrenergen Systems während des Nikotinentzuges berücksichtigt. Dadurch dass die Substanzen nicht subkutan, sondern intramuskulär verabreicht werden, können eine schnellere Anflutung sowie initial höhere Plasmaspiegel der anticholinergen und antinoradrenergen Substanzen realisiert werden, ohne dass es zu einer Gefährdung der Sicherheit des Patienten kommt.

Weiterhin ist es möglich, das pharmazeutisch wirksame Mittel in Form eines Nasensprays zu verabreichen. Die Dosierbereiche für die transnasale Applikation für Scopolamin und Atropin reichen von 0,05 bis 0,1 mg/Tag sowie für Clonidin von 0,05 bis 0,15 mg/Tag.

Die Anwendung transkutaner therapeutischer Systeme bzw. die Nutzung von Nasensprays ermöglicht eine sichere, kontinuierliche und komfortable Blockade der Entzugssymptomatik über Wochen und Monate. Die Verabreichung von Medikamenten über die Nasenschleimhaut besitzt verschiedene Vorteile. Sie erlaubt die einfache und sichere Handhabung eine Selbstmedikation durch den Patienten. Außerdem werden entscheidende Nachteile der oralen Medikamentengabe, wie die hepatische first-class Elimination oder mangelnde gastrointestinale Resorption vermieden (Chien YW, Chang SF, Crit Rev Ther Drug Carrier Syst 4 (1987) 67-194). Während die transnasale Applikationsform für Scopolamin bereits eine klinisch relevante Option zur Behandlung der Reisekrankheit darstellt (Klocker N et al., Eur J Pharm Sci 13 (2001) 227-232), erfolgt die Verabreichung von Clonidin bzw. Atropin über die Nasenschleimhaut bislang vorwiegend im Rahmen von Studien (Bahair SA et al., Res. Commun Chen Pathol Pharmacol 67 (1990) 241-248).

## Patentansprüche

1. Verwendung einer Wirkstoffkombination von Atropin, Scopolamin und Clonidin zur Herstellung eines Arzneimittels zum Nikotinentzug.

2. Verwendung des Arzneimittels nach Anspruch 1. eine Wirkstoffmenge von Clonidin zwischen 0,1 bis 0,5 mg sowie Atropin und Scopolamin von je 0,5 bis 1,5 mg enthaltend.

3. Verwendung des Arzneimittels nach Anspruch 1 oder 2 als Vakzine.

4. Verwendung des Arzneimittels nach Anspruch 1 oder 2 als Nasenspray.

5. Hautpflaster, **dadurch gekennzeichnet, dass** das Hautpflaster zwei separate Kontaktflächen aufweist, wobei eine Kontaktfläche zur Applikation von Clonidin und die zweite Kontaktfläche zur Applikation von Atropin und Scopolamin dient

## Claims

1. Use of a combination of active agents atropine, scopolamine and clonidine for producing a medicinal product for nicotine withdrawal.

2. Use of a medicinal product according to claim 1, containing an amount of clonidine between 0.1 to 0.5 mg as well as atropine and scopolamine each from 0.5 up to 1.5 mg as active agents.

3. Use of a medicinal product acccording to claim 1 or 2 as vaccine.

4. Use of a medicinal product according to claim 1 or 2 as nasal spray.

5. Dermal plaster, **characterized by** having two separate contact areas, whereas one contact area is used for the application of clonidine and the second contact area is used for the application of atropine and scopolamine.

## Revendications

1. Utilisation d'une combinaison de principes actifs d'atropine, de scopolamine et de clonidine pour fabriquer un médicament destiné au sevrage de la nicotine.

2. Utilisation du médicament selon la revendication 1, contenant une quantité de principe actif de clonidine comprise entre 0,1 et 0,5 mg, ainsi que d'atropine et de scopolamine comprise respectivement entre 0,5 et 1,5 mg.

3. Utilisation du médicament selon la revendication 1 ou 2 en tant que vaccin.

4. Utilisation du médicament selon la revendication 1 ou 2 en tant que spray nasal.

5. Timbre cutané, **caractérisé en ce que** le timbre cutané présente deux surfaces de contact séparées, une surface de contact servant à l'application de la clonidine et la seconde surface de contact à l'application de l'atropine et de la scopolamine.
